(19) Europäisches Patentamt
European Patent Office
Office européen des brevets

(11) Veröffentlichungsnummer : **0 363 758 B1**

# (12) EUROPÄISCHE PATENTSCHRIFT

(45) Veröffentlichungstag der Patentschrift :
**09.12.92 Patentblatt 92/50**

(51) Int. Cl.$^5$ : **C07C 217/78,** C08G 65/32, C08G 18/32, C08G 18/50, C08G 18/40

(21) Anmeldenummer : **89118111.7**

(22) Anmeldetag : **29.09.89**

## (54) Aromatische Polyhydroxypolyamine, ein Verfahren zu ihrer Herstellung und ihre Verwendung zur Herstellung von Polyurethan-Kunststoffen.

(30) Priorität : **12.10.88 DE 3834749**

(43) Veröffentlichungstag der Anmeldung :
**18.04.90 Patentblatt 90/16**

(45) Bekanntmachung des Hinweises auf die Patenterteilung :
**09.12.92 Patentblatt 92/50**

(84) Benannte Vertragsstaaten :
**AT BE CH DE ES FR GB IT LI NL SE**

(56) Entgegenhaltungen :
**EP-A- 0 252 351**
**EP-A- 0 268 849**
**EP-A- 0 288 825**
**US-A- 4 515 981**
**HOUBEN-WEYL: "METHODEN DER ORGANISCHEN CHEMIE" 4. Auflage, Band VI/1b, Alkohole III, Seiten 797,822, 1984,Georg-Thieme-Verlag, Stuttgart, DE**

(73) Patentinhaber : **BAYER AG**
**W-5090 Leverkusen 1 Bayerwerk (DE)**

(72) Erfinder : **Thiery, Urs, Dr.**
**Altstrasse 117**
**W-5090 Leverkusen 3 (DE)**
Erfinder : **Sanders, Josef, Dr.**
**2331 Norton Road**
**Pittsburgh, Pa 15241 (US)**
Erfinder : **Dieterich, Dieter, Prof. Dr.**
**Henry-Theodor-von-Böttinger-Strasse 16**
**W-5090 Leverkusen 1 (DE)**
Erfinder : **Grögler, Gerhard, Dr.**
**von-Diergardt-Strasse 48**
**W-5090 Leverkusen 1 (DE)**
Erfinder : **Reiff, Helmut, Dr.**
**Paul-Klee-Strasse 68a**
**W-5090 Leverkusen 1 (DE)**

EP 0 363 758 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue, endständige Aminophenoxy- und Hydroxygruppen aufweisende Verbindungen, ein Verfahren zu ihrer Herstellung und ihre Verwendung als Aufbaukomponente bei der Herstellung von gegebenenfalls zellförmigen Polyurethan-Kunststoffen und Schaumstoffen.

Endständige, aromatische Aminogruppen aufweisende Polyaddukte sind im Prinzip bekannt.

US-PS 2 888 439 und DE-OS 1 720 646 beschreiben die Herstellung von Aminopolyethern durch Reaktion von Nitroarylisocyanaten mit Polyolen und nachfolgender Hydrierung. Die analoge Umsetzung von Azoarylisocyanaten mit Polyolen liefert nach Reduktion ebenfalls aromatische Aminopolyether (DE-OS 1 257 427). In den DE-OS 1 122 254 und DE-OS 1 694 152 wird ein Verfahren beschrieben, bei dem NCO-Prepolymere mit Diaminen umgesetzt werden, welche unterschiedlich reaktive Aminogruppen aufweisen.

Auch die Reaktion von NCO-Prepolymeren mit Sulfaminsäure gemäß DE-AS 1 155 907, mit Ameisensäure gemäß FR-PS 1 415 317 oder mit Hydroxylgruppen aufweisenden Enaminen, Aldiminen oder Ketiminen gemäß DE-OS 2 116 882 sowie DE-OS 2 546 536 führt nach Hydrolyse bzw. Verseifung ebenso zu aromatischen Aminopolyethern, wie die thermische Spaltung von Urethanen aus NCO-Prepolymeren und sekundären oder tertiären Carbinolen gemäß DE-AS 1 270 046. Ferner beschreiben die DE-OS 2 948 419, 3 223 397, 3 223 398 sowie 3 223 400 verschiedene ein- bzw. zweistufige Verfahren zur Herstellung aromatischer Polyamine durch Hydrolyse von NCO-Prepolymeren in Gegenwart verschiedener Lösungsmittel- und Katalysatorsysteme.

Alle bisher beschriebenen Verfahren verlaufen über Isocyanatzwischenstufen und liefern daher in allen Fällen Produkte, die neben Ether- und aromatischen Aminogruppen zusätzliche Urethan- bzw. Harnstoffgruppen aufweisen. Dadurch bedingt zeigen diese Produkte eine für viele Zwecke unerwünscht hohe Viskosität. Ein weiterer Nachteil dieser zusätzlichen Urethan- bzw. Harnstoffbindungen ist ihre relativ geringe thermische Stabilität, wodurch der Wärmestand entsprechender mit diesen Aminopolyethern hergestellten Polyurethankunststoffe insbesondere von Elastomeren ungünstig beeinflußt wird.

Als weitere Synthesemöglichkeit für aromatische Polyamine kommt die unter Ringöffnung verlaufende Reaktion von Isatosäureanhydrid mit Polyolen in Betracht.

Solche Amine sind beispielsweise in den DE-OS 2 019 432, 2 619 840, 2 648 774, 2 648 825 sowie in US-PS 4 180 644, beschrieben. Aromatische Aminopolyether erhält man ferner durch Umsetzung von Polyoxyalkylenpolyolen mit p-Aminobenzoesäurederivaten gemäß den japanischen Patentanmeldungen 59 053 533, 59 089 322 und 59 199 715. Nachteilig für viele Zwecke ist jedoch die geringe Reaktivität der auf diesem Wege erhaltenen aromatischen Esteramine.

In der EP-A-268 849 wurden Polyphenoxyamine vorgestellt, welche günstigere Viskositäten und teilweise gute Reaktivitäten mit Isocyanaten aufzeigen. Bei der Darstellung der Polyphenoxynitroverbindungen, aus denen die Amine durch Hydrierung erhalten werden, verbleibt jedoch nicht umgesetztes Nitrophenylierungsmittel, das sich bei einer Verarbeitung der Amine äußerst störend auswirkt und zuvor aufwendig entfernt werden muß.

Es war daher Aufgabe der vorliegenden Erfindung, neue Polyhydroxypolyphenoxyamine zur Verfügung zu stellen, unter vollständigem Umsatz des Nitrophenylierungsmittels und insbesondere mit noch niedrigeren Viskositäten als oben genannte Polyphenoxyamine und gegebenenfalls günstigeren Reaktivitäten. Der durch die schnell reagierenden Aminogruppen und die langesamer reagierenden OH-Gruppen erreichte zweistufige Reaktionsverlauf wirkt sich besonders günstig z. B. beim Reaktionsspritzgießen aus. Durch die anfänglich niedrige Viskosität wird eine gute Vermischung im Mischkopf erreicht. Nach Reaktion der schnellen Aminogruppen steigt die Viskosität soweit an, daß Dichtungsprobleme an der Form nicht auftreten, wobei jedoch das Produkt lang genug dünnflüssig bleibt um die Form vollständig auszufüllen.

Diese Aufgabe konnte durch Bereitstellung der erfindungsgemäßen Verbindungen gelöst werden.

Gegenstand der vorliegenden Erfindung sind somit neue endständige Aminophenoxy- und Hydroxylgruppen aufweisende Verbindungen der allgemeinen Formel

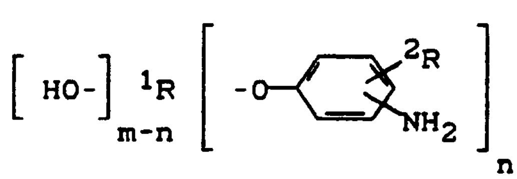

in welcher

$1_R$ für einen m-wertigen Rest steht, wie er durch teilweise Entfernung der Hydroxylgruppen von einer m-wertigen Polyhydroxylverbindung des Molgewichts 400 - 8000 entsteht,

$2_R$ für Wasserstoff oder eine Methylgruppe steht,

m eine ganze Zahl von 2 bis 4 bedeutet,

n ein Durchschnittswert und eine positive Zahl von 0,05·m bis 0,73·m bedeutet, vorzugsweise 0,15·m bis 0,70·m.

Gegenstand der vorliegenden Erfindung ist auch ein Verfahren zur Herstellung von endständige Hydroxy- und Aminophenoxygruppen aufweisenden Verbindungen der allgemeinen Formel

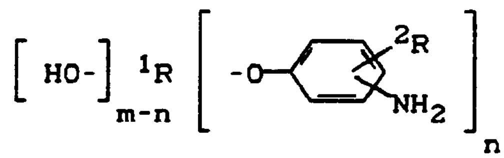

in der $^1R$, $^2R$, m und n die vorgenannte Bedeutung haben, dadurch gekennzeichnet, daß man

a)

(i) m-wertige höhermolekulare Polyhydroxylverbindungen der allgemeinen Formel

$$^1R(OH)_m$$

(ii) n-Molen der Verbindungen der allgemeinen Formel

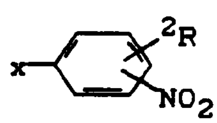

in Gegenwart von alkalisch reagierenden Verbindungen umsetzt und

b) die so erhaltenen (m-n)-Hydroxy, n-Nitrophenoxyaddukte der allgemeinen Formel

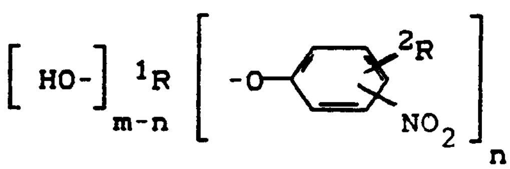

in an sich bekannter Weise zu den entsprechenden (m-n)-Hydroxy-, n-Aminophenoxy-Verbindungen hydriert, wobei in diesen Formeln $^1R$, $^2R$, m und n die in den Ansprüchen oben angegebene Bedeutung haben und x für Fluor oder Chlor steht.

Gegenstand der Erfindung ist schließlich auch die Verwendung der letztgenannten erfindungsgemäßen Hydroxy- und Aminophenoxygruppen aufweisenden Verbindungen als Aufbaukomponente bei der Herstellung von Polyurethankunststoffen nach dem Isocyanat-Polyadditionsverfahren.

Ausgangsmaterialien für das erfindungsgemäße Verfahren sind (i) höhermolekulare Polyhydroxylverbindungen und (ii) gegebenenfalls methylsubstituierte Nitrohalogenbenzole.

Geeignete Polyhydroylverbindungen (i) sind vorzugsweise höhermolekulare Verbindungen mit einem mittleren Molgewicht von 400 bis 8000, welche mindestens 2 bis 4 reaktive Hydroxylgruppen pro mol aufweisen.

In Frage kommen beispielsweise die in der Polyurethanchemie üblichen, hydroxylgruppentragenden Polyacetale, Polythioether, Polycarbonate, Polyamide, Polysiloxane und/oder Polybutadiene, Polyester, Polylactone sowie Polyether; insbesondere aber Hydroxylgruppen tragende Polyether.

Die erfindungsgemäß in Frage kommenden Hydroxylgruppen aufweisenden Polyether sind solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z. B . in Gegenwart von $BF_3$, oder durch Anlagerung dieser Expoxide, gegebenenfalls im Gemisch oder nacheinander, an Startkomponenten mit reaktionsfähigen Wasserstoffatomen wie Wasser, Alkohole oder Amine, z. B. Ethylenglykol, Propylenglykol-(1,3) oder -(1,2), Trimethylolpropan, 4,4′-Dihydroxy-diphenylpropan, Anilin, Ammoniak, Ethanolamin oder Ethylendiamin hergestellt. Auch Sucrosepolyether, wie sie z. B. in den deutschen Auslegeschriften 1 176 358 und 1 064 938 beschrieben werden, kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch durch Vinylpolymerisate modifizierte Polyether, wie sie z. B. durch Polymerisation von Styrol und Acrylnitril in Gegenwart von Polyethern entstehen (amerikanische Patentschriften 3 383 351, 3 304 273, 3 523 093, 3 110 659, deutsche Patentschrift 1 152 536), sind geeignet, ebenso OH-Gruppen aufweisende Polybutadiene.

Als Polyacetale kommen z. B. die aus Glykolen, wie Dioder Tri-ethylenglykol, 4,4′-Dihydroxyethoxydiphenylmethan, Hexandiol und Formaldehyd oder durch Polymerisation cyclischer Acetale, wie z. B. Trioxan, herstellbare Verbindungen in Frage.

Als Hydroxylgruppen aufweisende Polycarbonate kommen solche der an sich bekannten Art in Betracht, die z. B. durch Umsetzung von Diolen wie Propandiol-1,3, Butandiol-1,4 und/oder Hexandiol-1,6, Di-, Tri- oder Tetraethylenglykol oder Thiodiglykol mit Diarylcarbonaten, z. B. Diphenylcarbonat, oder Phosgen hergestellt werden können (DE-B 1 694 080, 1 915 908 und 2 221 751; DE-A 2 605 024).

Als Polyester aus Dicarbonsäuren und Diolen kommen solche aus Adipinsäure- und Isophthalsäure und geradkettigen und/oder verzweigten Diolen in Betracht, ebenso Lactonpolyester, vorzugsweise auf Basis von Caprolacton und Starterdiolen.

Unter den Polythioethern seien insbesondere die Kondensationsprodukte von Thiodiglykol mit sich selbst und/oder mit anderen Glykolen angeführt.

Auch bereits Urethan- oder Harnstoffgruppen enthaltende Polyhydroxylverbindungen sowie gegebenenfalls modifizierte natürliche Polyole sind verwendbar. Auch Anlagerungsprodukte von Alkylenoxiden an Phenol-Formaldehyd-Harze oder auch an Harnstoff-Formaldehyd-Harze sind erfindungsgemäß einsetzbar. Es ist auch möglich, z. B. gemäß DE-A 2 559 372, in die Polyhydroxylverbindungen Amidgruppen einzuführen.

Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den o. G., Hydroxylgruppen aufweisenden Verbindungen ablaufen läßt. Derartige Verfahren sind beispielsweise in den DE-B 1 168 075 und 1 260 142, sowie den DE-A 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-PS 3 869 413 bzw. 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z. B. durch Polymerisation von Styrol und Acrylnitril in Gegenwart von Polyethern (US-PS 3 383 351, 3 304 273, 3 523 093, 3 110 695, DE-B 1 152 536) oder Polycarbonatpolyolen (DE-PS 1 769 795, US-PS 3 637 909) erhalten werden, sind für das erfindungsgemäße Verfahren geeignet. Bei Verwendung von Polyetherpolyolen, welche gemäß DE-A 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit.

Beim Einsatz von modifizierten Polyhydroxylverbindungen der oben genannten Art als Ausgangsmaterialien für die Polyamine entstehen Ausgangskomponenten, die im Polyisocyanat-Polyadditionsverfahren in vielen Fällen Polyurethankunststoffe mit wesentlich verbesserten mechanischen Eigenschaften ergeben.

Geeignete, wenn auch weniger bevorzugte Polyhydroxylkomponenten (i) sind auch organofunktionelle Polysil oxane, die zwei endständige, gegenüber Isocyanatgruppen reaktionsfähige Gruppen und Struktureinheiten der Formel $-O-Si(R)_2-$ aufweisen, wobei in dieser Formel R für einen $C_1$-$C_4$-Alkyl- oder einen Phenylrest, vorzugsweise jedoch für einen Methylrest, steht. Erfindungsgemäß eignen sich als Ausgangsmaterialien sowohl die an sich bekannten, endständige organofunktionelle Gruppen aufweisenden reinen Polysiloxane als auch die an sich bekannten endständig organofunktionellen Siloxanpolyoxyalkylencopolymeren.

Die erfindungsgemäß besonders bevorzugten Organopolysiloxane entsprechen der allgemeinen Formel

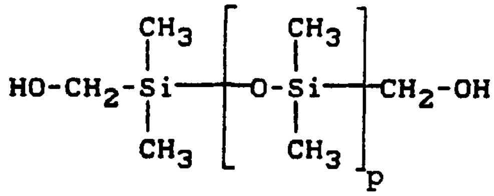

p = 5 bis 29

Sie werden in an sich bekannter Weise durch Äquilibrierung von 1,1,3,3-Tetramethyl-1,3-hydroxymethyl-disiloxan der Formel

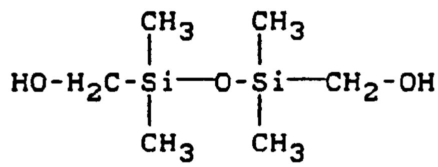

mit Octamethylcyclotetrasiloxan in Gegenwart von Schwefelsäure bzw. nach dem Verfahren der DE-B 1 236 505 hergestellt.

Zu den erfindungsgemäß in Betracht kommenden Ausgangsmaterialien (ii) gehören gegebenenfalls methylsubstituierte Halogennitrobenzole der allgemeinen Formel

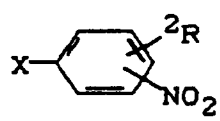

für welche

$^2R$ für Wasserstoff oder eine Methylgruppe, vorzugsweise für Wasserstoff steht,

X für Fluor oder vorzugsweise Chlor steht,

und in welcher die Halogen- und Nitrosubstituenten vorzugsweise ortho- oder paraständig zueinander angeordnet sind. In Betracht kommen beispielsweise 2-Nitrochlorbenzol, 2-Nitrofluorbenzol, 4-Nitrochlorbenzol, 4-Nitrofluorbenzol, 1-Methyl-2-nitro-3-chlorbenzol, 1-Methyl-2-nitro-3-fluorbenzol, 1-Methyl-4-nitro-5-chlorbenzol, 1-Methyl-4-nitro-5-fluorbenzol, 1-Methyl-2-nitro-6-chlorbenzol oder 1-Methyl-2-nitro-6-fluorbenzol. Besonders bevorzugte Ausgangsmaterialien (ii) sind 2-Nitrochlorbenzol oder 4-Nitrochlorbenzol.

Als alkalisch reagierende Verbindung, die zur Umsetzung der Polyhydroxylverbindungen (i) mit den Halogennitrobenzolen (ii) erforderlich sind, kommen beispielsweise Metallhydride, Metallalkoxide und bevorzugt Metallhydroxide in Frage. Besonders bevorzugt werden Natriumhydroxid und Kaliumhydroxid.

Bei der Durchführung der Stufe a) des erfindungsgemäßen Verfahrens werden die Ausgangsmaterialien (ii) bezogen auf die Komponente (i) im Unterschuß eingesetzt.

Der bei der Reaktion frei werdende Halogenwasserstoff kann, wie schon erwähnt, durch Zusatz von Metallhydriden, Metallalkoxiden und Metallhydroxiden gebunden werden. Bevorzugt werden Natrium- oder Kalium-hydroxid in fein verteilter Form eingesetzt. Dabei wird ihre Menge zumindest so bemessen, daß sie zur Neutralisation des abgespaltenen Chlorwasserstoffs ausreicht. Besonders bevorzugt werden sie in einer Menge verwendet, daß 1 bis 3 mol Basenäquivalente pro mol Komponenten (ii) zur Verfügung stehen.

Die Stufe a) des erfindungsgemäßen Verfahrens wird in Substanz oder zweckmäßig in einem organischen Lösungsmittel, gegegebenenfalls in Gegenwart eines Phasentransferkatalysators durchgeführt. Dabei können die Reaktionspartner in homogener Phase oder zweiphasig, gelöst, emulgiert oder suspendiert vorliegen.

Geeignete organische Lösungsmittel sind beispielsweise: Benzol, Toluol, Xylol, Chlorbenzol, Dichlorbenzol, Trichlorbenzol, Diethylether, Diisopropylether, tert.-Butylmethylether, Tetrahydrofuran, Dioxan, Ethylenglykoldimethylether, Essigester, Aceton, Methylethylketon, Acetonitril, Furfurol, Methylenchlorid, Chloroform, Trichlorethylen, Tetrachlorethylen, Nitromethan, Nitropropan, Dimethylformamid, Dimethylacetamid, N-Methylpyrrolidon, Tetramethylharnstoff, N-Methylcaprolactam, Dimethylsulfoxid, Tetramethylensulfon, Hexamethylenphosphorsäuretriamid usw.

Selbstverständlich können auch beliebige Gemische derartiger Lösungsmittel eingesetzt werden.

Die Menge des Lösungsmittels wird hierbei im allgemeinen so bemessen, daß sie ausreicht, um die Ausgangsmaterialien (i) und (ii) klar zu lösen. In der Praxis bedeutet dies, daß die Lösungsmittel im allgemeinen in einer Menge von 50 bis 1.000, bevorzugt 100 bis 500 Gew.-Teilen Lösungsmittel pro 100 Gew.-Teilen des Gemisches aus den Komponenten (i) und (ii) zum Einsatz gelangen.

Es kann in manchen Fällen vorteilhaft sein, die Umsetzung in Gegenwart eines Phasentransferkatalysators durchzuführen. Solche Katalysatoren werden z. B. in E. V. und S. S. Dehmlow, Phase Transfer Catalysis, 2. Auflage, Verlag Chemie 1983, beschrieben. Geeignete Katalysatoren sind quaternäre Ammonium- oder Phosphoniumsalze der Formel

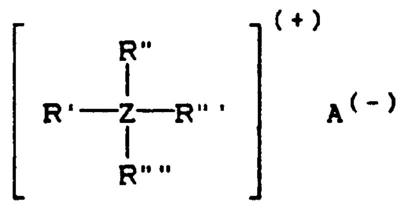

in welcher

Z für Stickstoff oder Phosphor steht,

R′, R″, R″ ′ und R″ ″ für gleiche oder verschiedene Reste stehen und Alkylgruppen mit 1 bis 18 Kohlenstoffatomen bedeuten, wobei einer der Reste auch für einen araliphatischen Rest mit 7 bis 16 Kohlenstoffatomen stehen kann, und wobei die Summe der Kohlenstoffatome der vier Reste vorzugsweise bei 12 bis 31 liegt.

Typische Beispiele geeigneter Katalysatoren sind N-Benzyl-N,N,N-triethyl-ammoniumchlorid oder -bromid, N-Benzyl-N-dodecyl-N,N-dimethyl-ammoniumchlorid oder -bromid, N,N,N,N-Tetra-n-hexyl-ammoniumchlorid oder -bromid, N-Benzyl-N,N,N-tri-n-octyl-ammoniumchlorid oder -bromid oder die diesen Ammoniumsalzen entsprechenden Phosphoniumsalze, vorzugsweise N,N,N-Trimethyl-N-benzylammoniumchlorid oder N-Hexadecyl-N,N,N-trimethylammoniumbromid.

Die beispielhaft genannten quarternären Ammonium- oder Phosphoniumsalze werden bei der Durchführung des erfindungsgemäßen Verfahrens vorzugsweise in Substanz oder in Form ihrer wäßrigen Lösungen (beispielsweise mit einem Feststoffgehalt von 30 bis 60 Gew.-%) und vorzugsweise in einer Menge von 1 bis 10 Mol-%, bezogen auf die Molzahl der vorhandenen Hydroxylgruppen eingesetzt.

Die Stufe a) des erfindungsgemäßen Verfahrens wird im allgemeinen bei 10 bis 100°C, bevorzugt bei 20 bis 80°C mit Überdruck, Unterdruck oder zweckmäßig drucklos, kontinuierlich oder diskontinuierlich durchgeführt.

Die Verweilzeit beträgt im allgemeinen 0,5 bis 24 Stunden; bevorzugt werden 0,5 bis 12 Stunden.

Zur Durchführung der Stufe a) des erfindungsgemäßen Verfahrens kann man beispielsweise so vorgehen, daß man die Ausgangsmaterialien und gegebenenfalls den Phasentransferkatalysator im gewählten Lösungsmittel vorlegt und die Base in gelöster oder suspendierter Form, bevorzugt in fester, möglichst fein gemahlener Form, unter Rühren, gegebenenfalls unter Kühlung portionsweise oder kontinuierlich zugibt. Anschließend rührt man bei Raumtemperatur oder gegebenenfalls erhöhter Temperatur so lange nach, bis dünnschichtchromatographisch ein vollständiger Umsatz des Nitrophenylierungsmittel (ii) angezeigt wird.

Die Aufarbeitung der Nitrophenoxyaddukte erfolgt in an sich bekannter Weise. Zweckmäßig verdünnt man das Reaktionsgemisch mit einem mit Wasser nicht mischbaren inerten Lösungsmittel, wäscht mit Wasser oder Salzlösung neutral, destilliert die Lösungsmittel gegebenenfalls im Vakuum ab und trocknet im Vakuum. Die Neutralisation des Reaktionsgemisches kann auch durch Behandeln mit $CO_2$ erfolgen. Als inerte Lösungsmittel kommen beispielsweise Tuluol, Methylenchlorid, Chlorbenzol, Dichlorbenzol, 1,2-Dichlorethan, Trichlorethylen u.a. in Betracht. Eine weitere Möglichkeit der Aufarbeitung besteht darin, daß nach Zusatz von 1-20 % Wasser, zum Reaktionsgemisch mit Mineralsäure, bevorzugt Salzsäure oder Schwefelsäure, gegegebenenfalls unter Vakuum, entfernt und die Mischung bei Temperaturen von 10-120°C, bevorzugt 25-90°C, filtriert. Das so erhaltene Rohprodukt kann im allgemeinen ohne weitere Reinigung weiterverarbeitet werden.

Grundsätzlich denkbar, jedoch weniger bevorzugt, wäre auch eine Arbeitsweise, die darin besteht, das in der Stufe a) anfallende Reaktionsgemisch, gegebenenfalls nach Neutralisation des überschüssigen Alkalihydroxids ohne Zwischenisolierung direkt der Stufe b) zuzuführen.

Die in der Stufe a) des erfindungsgemäßen Verfahrens erhaltenen endständige Nitrophenoxygruppen aufweisenden Verbindungen werden in der Stufe b) in an sich bekannter Weise durch Reduktion mit nascierendem oder katalytischem, beispielsweise mittels Raney-Nickel oder Palladium auf Kohle angeregtem Wasserstoff in die entsprechenden Polyamine überführt. Die Hydrierung kann in Gegenwart oder Abwesenheit von inerten Lösungsmitteln bei 20 - 120°C und einem Druck von 20 bis 80 bar erfolgen. Geeignete Lösungsmittel sind beispielsweise Methanol, Ethanol, i-Propanol, Toluol, DMF u. a. Bevorzugt wird Methanol oder Toluol. Die Diamine werden als Destillationsrückstand bei der destillativen Entfernung des Lösungsmittels gewonnen und können ohne weitere Reinigungsschritte zur Herstellung von Polyurethankunststoffen verwendet werden.

Die nach Aufarbeitung erhaltenen erfindungsgemäßen Polyhydroxypolyamine stellen im allgemeinen hellgelbe bis bräunlich gefärbte Produkte dar und zeichnen sich gegenüber den bisher bekannten aromatischen Aminopolyethern durch ihre niedrigere Viskosität aus. Sie erhalten neben den bereits in den zugrunde liegenden Polyhydroxylverbindungen vorhandenen funktionellen Gruppen wie z. B. Ether- und/oder Thioether- und/oder Dialkylsiloxan und/oder Carbonatgruppen und/oder Reste von Polybutadienen nur noch eine ihrer Funktionalität entsprechende Anzahl von Ethergruppen und Hydroxygruppen. Die erfindungsgemäßen aromatischen Polyhydroxypolyamine eignen sich als Reaktionspartner für gegebenenfalls blockierte Polyisocyanate bei der Herstellung von Polyurethanen (Polyurethanharnstoffen), gegebenenfalls zelligen Polyurethankunststoffen oder Polyurethanschaumstoffen, wobei sie gegebenenfalls auch mit anderen niedermolekularen (Molekulargewicht 32 bis 399) und/oder höhermolekularen (Molekulargewicht 400 bis ca. 12.000 Verbindungen mit gegenüber Isocyanaten reaktiven Gruppen kombiniert werden können. Geeignete Ausgangskomponenten für die Herstellung von Polyurethankunststoffen werden beispielsweise in DE-A 2 302 564, DE-A 2 432 764 (US-PS 3 903 679) sowie in den DE-A 2 639 083, 2 512 385, 2 513 815, 2 550 796, 2 550 797, 2 550 833, 2 550 860 und 2 550 862 genannt. Dort finden sich auch Hinweise auf bei der Polyurethanherstellung gegebenenfalls mitzuverwendenden Hilfs- und Zusatzstoffe.

Besonders geeignet sind die erfindungsgemäßen Polyhydroxypolyamine für eine Anwendung in Kombination mit festen Polyisocyanaten. Gemäß DE-A 3 230 757 können mit diesen Komponenten bei RT oder gegebenenfalls erhöhter Temperatur beliebig lang lagerfähige Reaktionssysteme hergestellt werden, die erst bei stärkerer Hitzeeinwirkung aushärten. Derartige Systeme werden allgemein als Einkomponentensysteme bezeichnet. Geeignete feste Polyisocyanate sind beispielsweise dimeres 2,4-Diisocyanatotoluol (TT) oder 3,3′-Dimethyl-4,4′-diisocyanatodiphenylharnstoff (TDIH).

Die Herstellung von Polyurethan(harnstoff)en mittels der erfindungsgemäß hergestellten Polyamine ist ebenfalls Gegenstand der vorliegenden Erfindung. Ihre Anwendung kann z. B. für Elastomere, Beschichtungen, Fäden in der Applikation aus Schmelzen, Lösungen, Dispersionen oder als Reaktivkomponentenmischung erfolgen. Weitere Verwendungszwecke der erfindungsgemäß hergestellten Polyamine sind z. B. Kupplungskomponenten für Diazofarbstoffe, Härter für Epoxid und Phenolharze, sowie alle anderen an sich bekannten Reaktionen von Aminen wie Amid- oder Imidbildung und andere.

Die Erfindung soll anhand der folgenden Beispiele näher erläutert werden (%-Angaben sind - soweit nicht anders vermerkt ist - Gew.-%).

a) Nitrophenylierung

<u>Beispiel 1</u>

1000 g (0,17 mol) eines entwässerten Trimethylolpropan (TMP) gestarteten Polypropylenoxid-(82,5 %)ethylenoxid-(17,5 %)-ethertriols der OH-Zahl 29, versetzt mit 26 g (0,16 mol) 4-Chlornitrobenzol, 14 g (0,25 mol) gepulverten Kaliumhydroxid und 9,25 g (0,05 mol) Trimethylbenzylammoniumchlorid werden 12 h bei 70°C gerührt. Bei Raumtemperatur werden 200 ml Wasser zugesetzt, die Mischung mit 50 %iger $H_2SO_4$ neutral gestellt, das Wasser abdestilliert und die anfallenden Salze abfiltriert.

Ausbeute: 800 g (79 % d.Th.)
Viskosität: 1500 mPas/26°C
OH-Zahl: 18
Gaschromatogramm: 0,0 % 4-Chlornitrobenzol

<u>Beispiel 2</u>

1000 g (0,17 mol) entwässertes Trimethylolpropan gestarteten Polypropylenoxid-(82,5 %)-ethylenoxid-(17,5 %)-ethertriol der OH-Zahl 29, 42 g (0,26 mol) 4-Chlornitrobenzol, 22,4 g (0,40 mol) Kaliumhydroxid und 9,25 g (0,05 mol) Trimethylbenzylammoniumchlorid werden analog Beispiel 1 umgesetzt.

Ausbeute: 840 g (81 5 d.Th.)

Viskosität: 1600 mPas/26°C
OH-Zahl: 14
Gascromatogramm: 0,0 % 4-Chlornitrobenzol

Beispiel 3

1000 g (0,17 mol) entwässertes TMP gestarteten Polypropylenoxid-(82,5 %)-ethylenoxid-(17,5 %)-ethertriol der OH-Zahl 29, 57,5 g (0,36 mol) 4-Chlornitrobenzo, 30,8 g (0,55 mol) Kaliumhydroxid und 9,25 g (0,05 mol) Trimethylbenzylammoniumchlorid werden analog Beispiel 1 umgesetzt.
Ausbeute: 820 g (78 % d.Th.)
Viskosität: 1900 mPas/26°C
OH-Zahl: 9
Gascrhomatogramm: 0,0 % 4-Chlornitrobenzol

Beispiel 4

1000 g (0,17 mol) eines entwässerten TMP gestarteten Polypropylenoxid-(82,5 %)-ethylenoxid-(17,5 %)-ethertriols der OH-Zahl 29, gelöst in 1000 ml Toluol, werden mit 26 g (0,16 mol) 4-Chlornitrobenzol, 14 g (0,25 mol) Kaliumhydroxid und 9,25 g (0,05 ml) Trimethylbenzylammoniumchlorid versetzt und 12 h bei 70°C gerührt.
Bei Raumtemperatur werden 200 ml Wasser zugesetzt und die Mischung mit 50 %iger $H_2SO_4$ neutral gestellt. Anschließend wird das Lösungsmittel und das Wasser abdestilliert und die ausfallenden Salze abfiltriert.
Ausbeute: 898 g (88 % d.Th.)
Viskosität: 1500 mPas/20°C
OH-Zahl: 18
Gaschromatogramm: 0,0 % 4-Chlornitrobenzol

Beispiel 5

1000 g (0,17 mol) entwässertes TMP gestarteten Polypropylenoxid-(82,5 %)-ethylenoxid-(17,5 %)-ethertriol der OH-Zahl 29 gelöst in 1000 ml Toluol, 42 g (0,26 mol) 4-Chlornitrobenzol, 22,4 g (0,40 mol) Kaliumhydroxid und 9,25 g (0,05 % mol) Trimethylbenzylammoniumchlorid werden analog Beispiel 4 umgesetzt.
Ausbeute: 955 g (92 % d.Th.)
Viskosität: 1400 mPas/27°C
OH-Zahl: 14
Gaschromatogramm: 0,0 % 4-Chlornitrobenzol

Beispiel 6

1000 g (0,17 mol) entwässertes TMP gestarteten Polypropylenoxid-(82,5 %)-ethylenoxid-(17,5 %)-ethertriol der OH-Zahl 29 gelöst in 1000 ml Toluol, 57,5 g (0,36 mol) 4-Chlornitrobenzol, 30,8 g (0,55 mol) Kaliumhydroxid und 9,25 g (0,05 mol) Trimethylbenzylammoniumchlorid werden analog Beispiel 4 umgesetzt.
Ausbeute: 1028 g (100 % d.Th.)
Viskosität: 1700 mPas/20°C
OH-Zahl: 9
Gaschromatogramm: 0,1 % 4-Chlornitrobenzol

Beispiel 7

1400 g (1 mol) entwässertes Polyethylenoxidetherdiol der OH-Zahl 80 gelöst in 1500 ml Toluol, werden mit 167 g (1,06 mol) 4-Chlornitrobenzol, 90 g (1,6 mol) Kaliumhydroxid und 11,1 g (0,06 mol) Trimethylbenzylammoniumchlorid versetzt und 12 h bei 70°C gerührt.
Bei Raumtemperatur werden 300 ml Wasser zugesetzt und mit 50%iger $H_2SO_4$ neutral gestellt. Anschließend wird das Lösungsmittel und das Wasser abdestilliert und die ausfallenden Salze abfiltriert.
Ausbeute: 1440 g (94 % d.Th.)
Viskosität: 140 mPas/70°C
OH-Zahl: 35
Gaschromatogramm: 0,0% 4-Chlornitrobenzol

Beispiel 8

1000 g (0,17 mol) eines entwässerten TMP gestarteten Polypropylenoxid-(82,5 %)-ethylenoxid-(17,5 %)-ethertriols der OH-Zahl 29 werden mit 57,5 g (0,36 mol) 4-Chlornitrobenzol und 30,8 g (0,55 mol) Kaliumhydroxid versetzt und 12 h unter Durchleiten von Luft bei 70°C gerührt. Bei Raumtemperatur werden 200 ml Wasser zugesetzt und mit 50 %iger $H_2SO_4$ neutral gestellt. Anschließend wird das Wasser abdestilliert und die ausfallenden Salze abfiltriert.

Ausbeute: 756 g (74 % d.Th.)
Viskosität: 2000 mPas/26°C
OH-Zahl: 10
Gaschromatogramm: 0,0 % 4-Chlornitrobenzol

Beispiel 9

1000 g (0,17 mol) eines entwässerten TMP gest. Polypropylenoxid-(82,5 %)ethylenoxid-(17,2 %)-ethertriols der OH-Zahl 29 wurden it 42 g (0,26 mol) 2-Chlornitrobenzol, 22,4 g (0,40 mol) Trimethylbenzylammoniumchlorid versetzt und 12 h unter Durchleiten von Luft bei 70°C gerührt.

Bei Raumtemperatur werden 200 ml Wasser zugesetzt und mit 50%iger $H_2SO_4$ neutral gestellt. Anschließend wird das Wasser abdestilliert und die ausfallende Salze abfiltriert.

Ausbeute: 770 g (75 % d.Th.)
Viskosität: 2500 mPas/26°C
OH-Zahl: 16
Gaschromatogramm: 0,0 % 2-Chlornitrobenzol

Beispiel 10

1000 g (0,21 mol) eines TMP gestarteten Polypropylenoxid-(82,5 %)-ethylenoxid-(17,2 %)-ethertriols der OH-Zahl 35 wurden mit 105 g (0,94 mol) 50 %iger wäßriger Kaliumhydroxidlösung und 100 ml Toluol versetzt. Die Wasser/Toluol-Mischung wirde durch Vakuumdestillation (70°C/20 mbar) entfernt.

Der entwässerte Polyether wurde mit 69 g (0,44 mol) p-Chlornitrobenzol, gelöst in 170 ml Toluol, versetzt und unter Vakuum (20 mbar) 17 h bei 40°C gerührt. Nach Zugabe von 115 ml Wasser zu Reaktionsmischungstellte man mit 50 %iger Schwefelsäure neutral. Anschließend wurde das Wasser abdestilliert und die ausgefallenen Salze bei 80°C abfiltriert.

Ausbeute: 940 g (88 % d.Th.)
Viskosität: 1600 mPas/24°C
OH-Zahl: 12
Gel: 0,0 % 4-Chlornitrobenzol

b) Hydrierung

Allgemeine Vorschrift (für Beispiel 11-18)

100 Teile Polyhydroxypolynitroverbindung werden in 100 Teilen Lösungsmittel gelöst und mit 10 Teilen Raney-Nickel versetzt. Die Hydrierung erfolgt in einem Druckkessel bei einem Wasserstoffdruck von 60 bar zu Beginn der Reaktion und einer Temperatur von 70°C.

Die Daten der Beispiele 11 bis 18 sind Tabelle 1 zu entnehmen.

**Tabelle 1**

| Polyhydroxy-polynitroverb. | | | | | → | Polyhydroxy-polyamin | | | |
|---|---|---|---|---|---|---|---|---|---|
| Beispiel | $\overline{MG}$ | f(-OH)) | f(-$NO_2$) | OH-Zahl | | Beispiel | OH-Zahl | NH-Zahl | Viskosität [mPas/25°C] |
| 1 | 6121 | 2 | 1 | 18 | | 11 | 30 | 9 | 1700 |
| 2 | 6191 | 1,4 | 1,6 | 14 | | 12 | 30 | 14 | 2000 |
| 3 | 6266 | 0,8 | 2,2 | 9 | | 13 | 30 | 21 | 2300 |
| 5 | 6193 | 1,4 | 1,6 | 14 | | 14 | 30 | 18 | 1800 |
| 6 | 6266 | 0,8 | 2,2 | 9 | | 15 | 29 | 22 | 2000 |
| 8 | 6266 | 0,8 | 2,2 | 10 | | 16 | 29 | 22 | 2000 |
| 9 | 6193 | 1,4 | 1,6 | 16 | | 17 | 30 | 14 | 1600 |
| 10 | 5054 | 0,9 | 2,1 | 12 | | 18 | 35 | 24 | 1700 |

MG: durchschnittliches Molekulargewicht

OH-Zahl und NH-Zahl in mg KOH/g Verbindung

c) Anwendungsbeispiele

Beispiel 19

200 g des in Beispiel 15 beschriebenen Aminopolyethers der Aminzahl 22 werden 15 Minuten bei Raumtemperatur im Wasserstrahlvakuum entgast und mit 19,0 g feingemahlenem dimeren 2,4-Diisocyanat (TT) der

mittleren Korngröße 10 bis 30 µ versetzt. Anschließend wird durch kurzzeitiges intensives Rühren (ca. 1 Minute) eine möglichst feinteilige Suspension hergestellt und innerhalb von 2 bis 3 Minuten in eine mit Trennmittel behandelte, auf 100°C vorgeheizte Form gegossen. Nach ca. 30 bis 60 Minuten bei 120-130°C wird der inzwischen verfestigte Prüfkörper entformt und weitere 3 bis 4 Stunden bei dieser Temperatur getempert.

| | |
|---|---|
| Reißfestigkeit: | 6,0 kp/abs |
| Reißdehnung: | 250 % |
| Modul 100 %: | 3,16 mPa |
| Weiterreißwiderstand: | 81 N/cm |
| Shore Härte A: | 74 |
| Elastizität: | 46 % |

d) Vergleichsbeispiel mit vollständiger Nitrophenylierung

Beispiel 20

400 g (0,07 mol) eines entwässerten Polypropylenoxidethertriols der OH-Zahl 29, versetzt mit 30 g (0,19 mol) 4-Chlornitrobenzol, 16,8 (0,3 mol) Kaliumhydroxid und 3,7 g (0,02 mol) Trimethylbenzylammoniumchlorid werden 22 h bei 70°C gerührt. Bei Raumtemperatur werden 100 ml Wasser zugesetzt, die Mischung mit 50 %iger $H_2SO_4$ neutral gestellt, das Wasser abdestilliert und die anfallenden Salze abfiltriert.

| | |
|---|---|
| Ausbeute: | 383 g (91 % d.Th.) |
| Viskosität: | 2000 mPas/26°C |
| OH-Zahl: | 5 |
| Gaschromatogramm: | 1,0 % 4-Chlornitrobenzol |

Aus dem Vergleichsbeispiel 20 ist ersichtlich, daß bei geringem Unterschuß an Nitrophenylierungsmittel (4-Chlornitrobenzol) ein Rest verbleibt (1,0 % 4-Chlornitrobenzol), der vor der weiteren Umsetzung aufwendig entfernt werden muß.

e) Anwendungsgegenbeispiel

Beispiel 21

Analog Beispiel 19 werden 200 g Aminopolyether, erhalten durch Hydrierung von Nitroverbindung analog Beispiel 20 nach vorheriger Entfernung des Restgehalts an 4-Chlornitrobenzol, der Aminzahl 26 mit 19,0 g feingemahlenem dimeren 2,4-Diisocyanat (TT) der mittleren Korngröße 10 bis 30 µ umgesetzt.

| | |
|---|---|
| Reißfestigkeit: | 6,9 kp/abs |
| Reißdehnung: | 250 % |
| Modul 100 %: | 5,2 mPa |
| Weiterreißwiderstand: | 78 N/cm |
| Shore Härte A: | 79 |
| Elastizität: | 47 % |

Das Anwendungsgegenbeispiel 21 zeigt, daß bei fast vollständiger Aminierung des Polyols kein wesentlich besseres Werteniveau der daraus hergestellten Kunststoffe erreicht wird.

## Patentansprüche

1. 1. Endständige Aminophenoxy- und Hydroxylgruppen aufweisende Verbindungen der allgemeinen Formel

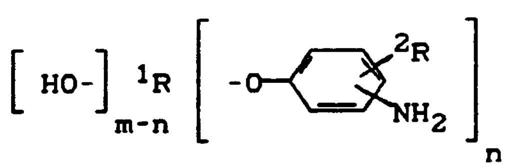

in welcher

$^1R$ für einen m-wertigen Rest steht, wie er durch teilweise Entfernung der Hydroxylgruppen von

einer m-wertigen Polyhydroxylverbindung des Molgewichts 400 - 8000 entsteht,

$2_R$ für Wasserstoff oder eine Methylgruppe steht,

m eine ganze Zahl von 2 bis 4 bedeutet,

n ein Durchschnittswert und eine positive Zahl von 0,05·m bis 0,73·m bedeutet, vorzugsweise 0,15·m bis 0,70·m.

2. Aromatische Polyhydroxypolyamine nach Anspruch 1, dadurch gekennzeichnet, daß $^1R$ für einen Polyalkylenpolyetherrest steht.

3. Aromatische Polyhydroxypolyamine nach einem der Ansprüche 1 oder 2, dadurch gekennzeichnet, daß $^2R$ für Wasserstoff steht und der Ethersauerstoff und die Aminogruppe ortho- oder paraständig zueinander angeordnet sind.

4. Aromatische Polyhydroxypolyamine nach einem der Ansprüche 1 bis 3, dadurch gekennzeichnet, daß n ein Durchschnittswert und eine positive Zahl von 0,3·m bis 0,6·m bedeutet.

5. Aromatische Polyhydroxypolyamine nach einem der Ansprüche 1 bis 4, dadurch gekennzeichnet, daß m einen Durchschnittswert von 2 bis 3 bedeutet.

6. Verfahren zur Herstellung von Polyhydroxyaminen nach einem der Ansprüche 1 bis 5, dadurch gekennzeichnet, daß man

a)

(i) m-wertige höhermolekulare Polyhydroxylverbindungen der allgemeinen Formel

$^1R(OH)_m$ mit

(ii) n-Molen der Verbindungen der allgemeinen Formel

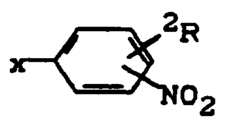

in Gegenwart von alkalisch reagierenden Verbindungen umsetzt und

b) die so erhaltenen (m-n)-Hydroxy, n-Nitrophenoxyaddukte der allgemeinen Formel

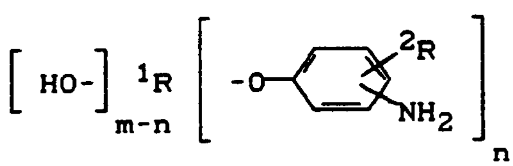

in an sich bekannter Weise zu den entsprechenden (m-n)-Hydroxy-, n-Aminophenoxy-Verbindungen hydriert, wobei in diesen Formeln $^1R$, $^2R$, m und n die in den Ansprüchen 1 bis 5 angegebene Bedeutung haben und x für Fluor oder Chlor steht.

7. Verfahren nach Anspruch 6, dadurch gekennzeichnet, daß man die Stufe a) des Verfahrens in Gegenwart von gepulvertem Natrium- und/oder Kaliumhydroxid in einer zur Neutralisation des abgespaltenen Halogenwasserstoffs mindestens ausreichenden Menge und gegebenenfalls mit Lösungsmittel und gegebenenfalls mit Phasentransferkatalysator durchführt.

8. Verfahren nach einem der Ansprüche 6 oder 7, dadurch gekennzeichnet, daß man die Stufe a) des Verfahrens lösungsmittelfrei durchführt.

9. Verfahren nach einem der Ansprüche 6 bis 8, dadurch gekennzeichnet, daß man die Stufe a) des Verfahrens in Gegenwart von DMSO oder aromatischen Kohlenwasserstoffen durchführt.

10. Verfahren nach einem der Ansprüche 6 bis 9, dadurch gekennzeichnet, daß man die Stufe a) des Verfahrens in Gegenwart von Trimethylbenzylammoniumchlorid, Triethylbenzylammoniumchlorid oder

Hexadecyltrimethylammoniumbromid durchführt.

11. Verfahren nach einem der Ansprüche 6 bis 10, dadurch gekennzeichnet, daß x für Chlor steht.

12. Verwendung der Polyhydroxypolyamine nach einem der Ansprüche 1 bis 5 als Aufbaukomponenten bei der Herstellung von Polyurethankunststoffen nach dem Isocyanat-Polyadditionsverfahren.

## Claims

1. Aminophenoxy- and hydroxyl-terminated compounds corresponding to the following general formula

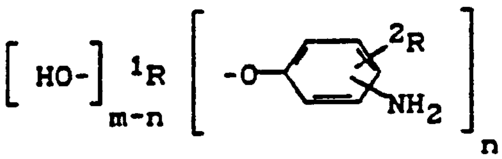

in which

$^1R$ is an m-functional residue of the type formed by partial removal of the hydroxyl groups from an m-functional polyhydroxyl compound having a molecular weight of 400 to 8000,

$^2R$ is hydrogen or a methyl group,

m is an integer of 2 to 4,

n is an average value and stands for a positive number of $0.05 \cdot m$ to $0.73 \cdot m$ and preferably of $0.15 \cdot m$ to $0.70 \cdot m$.

2. Aromatic polyhydroxypolyamines as claimed in claim 1, characterized in that $^1R$ is a polyalkylene polyether residue.

3. Aromatic polyhydroxypolyamines as claimed in claim 1 or 2, characterized in that $^2R$ is hydrogen and the ether oxygen and the amino group are in the ortho- or paraposition to one another.

4. Aromatic polyhydroxypolyamines as claimed in any of claims 1 to 3, characterized in that n is an average value and stands for a positive number of from $0.3 \cdot m$ to $0.6 \cdot m$.

5. Aromatic polyhydroxypolyamines as claimed in any of claims 1 to 4, characterized in that m is an average value of 2 to 3.

6. A process for the production of the polyhydroxyamines claimed in any of claims 1 to 5, characterized in that

a)

(i) m-functional, relatively high molecular weight polyhydroxyl compounds corresponding to the following general formula

$$^1R(OH)_m$$

are reacted with

(ii) n-mol of compounds corresponding to the following general formula

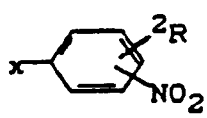

in the presence of compounds showing an alkaline reaction and

b) the (m-n)-hydroxy, n-nitrophenoxy adducts thus obtained, which correspond to the general formula

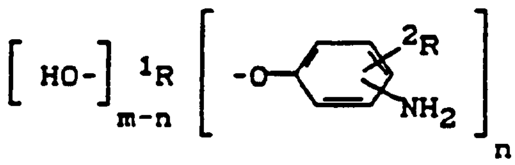

are hydrogenated in known manner to the corresponding (m-n)-hydroxy, n-aminophenoxy compounds; in the above formulae $^1R$, $^2R$, m and n have the meanings defined in claims 1 to 5 and x is fluorine or chlorine.

7. A process as claimed in claim 6, characterized in that step a) of the process is carried out in the presence of powdered sodium and/or potassium hydroxide in a quantity at least sufficient to neutralize the hydrogen halide given off and optionally with solvent and optionally with phase transfer catalyst.

8. A process as claimed in claim 6 or 7, characterized in that step a) of the process is carried out in the absence of a solvent.

9. A process as claimed in any of claims 6 to 8, characterized in that step a) of the process is carried out in the presence of DMSO or aromatic hydrocarbons.

10. A process as claimed in any of claims 6 to 9, characterized in that step a) of the process is carried out in the presence of trimethyl benzyl ammonium chloride, triethyl benzyl ammonium chloride or hexadecyl trimethyl ammonium bromide.

11. A process as claimed in any of claims 6 to 10, characterized in that x is chlorine.

12. The use of the polyhydroxypolyamines claimed in any of claims 1 to 5 as synthesis components in the production of polyurethane plastics by the isocyanate polyaddition process.

**Revendications**

1. Composés présentant des groupes aminophénoxy et hydroxyle terminaux, répondant à la formule générale

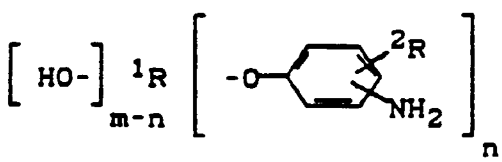

dans laquelle

$^1R$ représente un radical à valence m, tel qu'on l'obtient par élimination partielle des groupes hydroxyle d'un composé polyhydroxylé à valence m ayant un poids molaire de 400 à 8.000,

$^2R$ représente un atome d'hydrogène ou un groupe méthyle,

m représente un entier de 2 à 4,

n représente une valeur moyenne et un nombre positif de 0,05·m à 0,73·m, de préférence de 0,15·m à 0,70·m.

2. Polyhydroxypolyamines aromatiques selon la revendication 1, caractérisées en ce que $^1R$ représente un radical polyalkylène-polyéther.

3. Polyhydroxypolyamines aromatiques selon l'une quelconque des revendications 1 et 2, caractérisées en ce que $^2R$ représente un atome d'hydrogène, l'oxygène du groupe éther et le groupe amino étant disposés en position ortho ou para l'un par rapport à l'autre.

4. Polyhydroxypolyamines aromatiques selon l'une quelconque des revendications 1 à 3, caractérisées en

ce que n représente une valeur moyenne et un nombre positif de 0,3·m à 0,6·m.

**5.** Polyhydroxypolyamines aromatiques selon l'une quelconque des revendications 1 à 4, caractérisées en ce que m représente une valeur moyenne de 2 ou 3.

**6.** Procédé pour la préparation de polyhydroxyamines selon l'une quelconque des revendications 1 à 5, caractérisé en ce qu'on fait réagir

a)

(i) des composés polyhydroxylés à poids moléculaire élevé et à valence m, répondant à la formule générale

$^{1}R(OH)_m$ avec

(ii) n moles des composés répondant à la formule générale

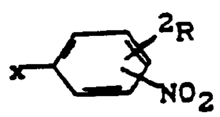

en présence de composés à réaction alcaline et

b) on soumet à une hydrogénation les adduits (m-n)-hydroxy, n-nitrophénoxy ainsi obtenus répondant à la formule générale

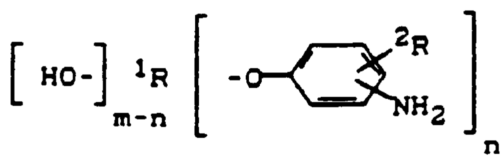

d'une manière connue en soi pour obtenir les composés (m-n)-hydroxy-, n-aminophénoxy correspondants; dans ces formules, $^{1}R$, $^{2}R$, m et n ont la signification indiquée ci-dessus dans les revendications 1 à 5 et x représente un atome de fluor ou un atome de chlore.

**7.** Procédé selon la revendication 6, caractérisé en ce qu'on effectue l'étape a) du procédé en présence d'hydroxyde de sodium et/ou de potassium pulvérulent en une quantité au moins suffisante pour la neutralisation de l'hydracide halogéné séparé et éventuellement avec un solvant et éventuellement avec un catalyseur de transfert de phase.

**8.** Procédé selon l'une quelconque des revendications 6 ou 7, caractérisé en ce qu'on effectue l'étape a) du procédé sans solvant.

**9.** Procédé selon l'une quelconque des revendications 6 à 8, caractérisé en ce qu'on effectue l'étape a) du procédé en présence de DMSO ou d'hydrocarbures aromatiques.

**10.** Procédé selon l'une quelconque des revendications 6 à 9, caractérisé en ce qu'on effectue l'étape a) du procédé en présence de chlorure de triméthylbenzylammonium, de chlorure de triéthylbenzylammonium ou encore de bromure d'hexadécyltriméthylammonium.

**11.** Procédé selon l'une quelconque des revendications 6 à 10, caractérisé en ce que x représente un atome de chlore.

**12.** Utilisation des polyhydroxypolyamines selon l'une quelconque des revendications 1 à 5 comme constituants lors de la préparation de matières synthétiques en polyuréthanne selon le procédé de polyaddition d'isocyanate.